# EUROPEAN PATENT APPLICATION

(11) **EP 1 127 585 A2**
(43) Date of publication of application: **29.08.2001**
(21) Application number: 01301738.9
(22) Date of filing: 26.02.2001
(51) Int. Cl.: A61N 1/16

(54) **System and method for protecting against geopathic radiation**

(30) Priority: 25.02.2000 US 185004 P
(71) Applicant: Leightner, Paul E., Pisgah Forest, North Carolina 28768 (US); Leightner, Daniel E., Allen, Texas 75002 (US)
(72) Inventor: Leightner, Paul E., Pisgah Forest, North Carolina 28768 (US); Leightner, Daniel E., Allen, Texas 75002 (US)
(74) Representative: Lawrence, John

(57) **Abstract**

A method of blocking geopathic radiation (54) is provided that includes identifying a location (10;50) at which the blocking of geopathic radiation (54) is desired. The method further includes positioning a layer of material including mica (20;42;52) between a source of the geopathic radiation and the identified location, such that the geopathic radiation is at least partially prevented from being transmitted to the identified location (10;50).

## Description

### TECHNICAL FIELD OF THE INVENTION

This invention relates generally to the field of radiation shielding, and more specifically to a system and method for blocking geopathic radiation.

### BACKGROUND OF THE INVENTION

Geopathic radiation is an ultra high frequency radiation originating in the core of the earth. The geopathic radiation filters out through the crust of the earth and occurs in varying intensities at the earth's surface. The presence of this radiation, especially in a location where a person spends a significant amount of time, produces illnesses and maladies including arthritis, cancer, and sleep disturbances. This radiation also contributes to a vast array of other illnesses by interfering with the immune system and the production of growth hormones which rebuild the body during sleep. Despite the many detrimental effects of geopathic radiation, little has been done to shield people from this type of radiation.

U.S. Patent No. 5,153,378, entitled Personal Space Shielding Apparatus, discloses an invention that purports to shield a person from electromagnetic radiation, including geopathic radiation. This patent discloses the use of shields that are made of ferrous, wire mesh screening that are erected around a personal occupancy space to protect the occupant from this radiation. However, studies have shown that the use of ferrous metal screens is ineffective in blocking geopathic radiation.

Mica is a substance that is conventionally used for electrical insulation. Mica has never before been used, to the knowledge of the inventors, in any system or method for blocking geopathic radiation.

### SUMMARY OF THE INVENTION

In accordance with the present invention, a system and method for blocking geopathic radiation is provided that substantially eliminates or reduces disadvantages or problems associated with previously developed systems and methods. In particular, the present invention contemplates using mica in appropriate forms to block or otherwise impede the transmission of geopathic radiation.

In one embodiment of the present invention, a method of blocking geopathic radiation is provided that includes identifying a location at which the blocking of geopathic radiation is desired. The method further includes positioning a layer of material including mica between a source of the geopathic radiation and the identified location, such that the geopathic radiation is at least partially prevented from being transmitted to the identified location.

In another embodiment of the present invention, a building foundation is provided that includes a quantity of concrete sufficient to structurally support a building that is to be supported by the foundation. The building foundation also includes a quantity of mica mixed into the concrete. In a particular embodiment, the quantity of mica mixed into the concrete comprises a quantity of mica sufficient to at least partially prevent geopathic radiation from being transmitted from a source of geopathic radiation to the building.

Technical advantages of the present invention include a system and method for blocking geopathic radiation so as to help to alleviate problems resulting from exposure to this radiation. By employing embodiments of the present invention to block geopathic radiation, a person can avoid or substantially avoid the adverse effects of this radiation and thereby improve overall health, as well as the quality and length of life.

Other technical advantages are readily apparent to one skilled in the art from the following figures, descriptions, and claims.

### BRIEF DESCRIPTION OF THE DRAWINGS

For a more complete understanding of the present invention, and for further features and advantages, reference is now made to the following description, taken in conjunction with the accompanying drawings, in which:
FIGURE 1 illustrates several embodiments of the present invention employed in a room of a building to prevent or partially prevent geopathic radiation from being transmitted to certain shielded areas of the room; and
FIGURE 2 illustrates a building having a foundation that is operable to block geopathic radiation.

### DETAILED DESCRIPTION OF THE INVENTION

The earth has a natural magnetic field formed by the rotation of the earth. This rotation creates electrical currents in the molten iron found within the earth's core, thereby producing a magnetic field. In addition to this magnetic field, the earth's core also produces a range of electromagnetic radiation. One type of electromagnetic radiation produced is an ultra-high frequency radiation typically referred to as geopathic radiation. Geopathic radiation filters out from the core of the earth and occurs in varying intensities at the surface of the earth. These varying intensities are caused by disturbances under the surface of the earth. These disturbances (including geological faults, underground ore masses, underground water, and mineral deposits, among others) reflect, refract, and/or diffuse the geopathic radiation as it travels through the earth and thereby concentrate the geopathic radiation at certain areas on the surface of the earth.

Studies have shown that the presence of geopathic radiation, especially in a location where a person spends a significant amount of time, produces illnesses and maladies including arthritis, cancer, and sleep disturbances. Geopathic radiation can contribute to a vast array of other illnesses by interfering with the immune system and the production of growth hormones which rebuild the body during sleep. These studies, several of which have been funded by European governments, attest to the existence of geopathic radiation and document evidence of the illnesses that this radiation induces. These studies also evidence immediate relief from symptoms of certain illnesses when the patient is removed from the area of radiation. Because of the observed detrimental effects of geopathic radiation, certain municipalities in Europe require a determination of the intensity of the geopathic radiation at a location before a building permit is issued.

One study has found that these detrimental effects may be due to the effect of geopathic radiation on the surface tension of water. Geopathic radiation has been shown to change this surface tension, and since humans are over 70% water, this change in surface tension greatly effects the operation of the human body (for example, the membrane process of creating seratonin, which regulates sleep). The effect of geopathic radiation on the human body is discussed in further detail in Geopathic Stress: How Earth Energies Affect Our Lives, by Jane Thurnell-Read, Element Books (Rockport, MA), Copyright 1995, which is incorporated herein by reference.

While one method of avoiding the detrimental effects of geopathic radiation is to avoid the radiation altogether, this is often impossible due to the fact that one or more zones of geopathic radiation may be pervasive throughout an existing building or other occupied areas. Furthermore, geopathic radiation zones may shift over time, making it difficult to move away from the radiation.

Embodiments of the present invention solve this problem by providing geopathic radiation shields that may be positioned between a source of geopathic radiation and an area to be protected or shielded (hereinafter referred to as the "shielded area"). In order to protect a shielded area from geopathic radiation, embodiments of the present invention employ mica, which has been discovered to prevent the transmission of geopathic radiation, in a number of different forms.

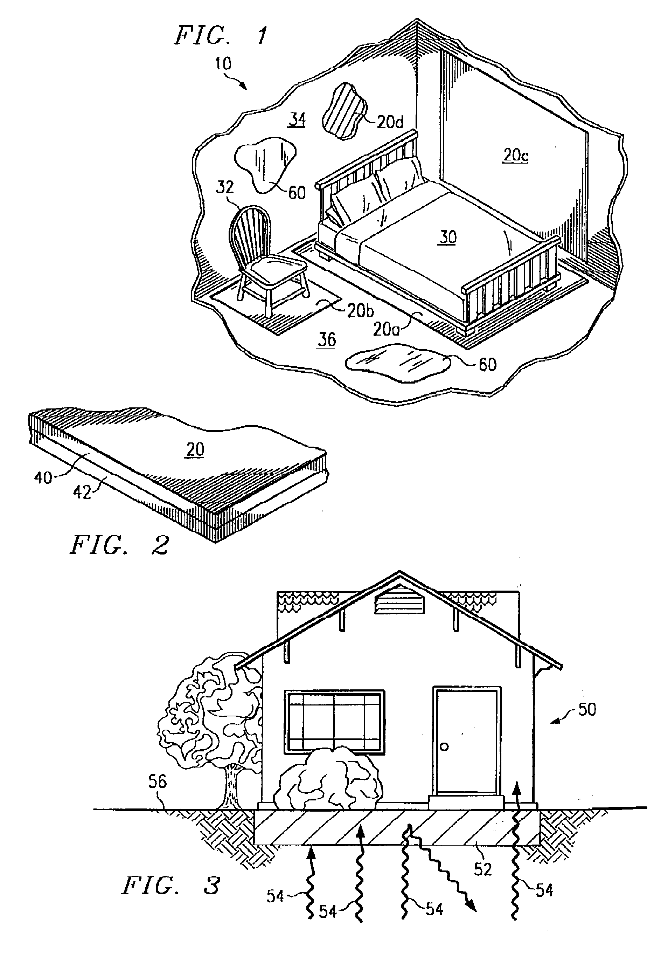

FIGURE 1 illustrates several embodiments of the present invention employed in a room 10 of a building to prevent or partially prevent geopathic radiation from being transmitted to certain shielded areas of room 10. In the illustrated embodiment, sheets 20 of mica (or material loaded or doped with mica) are used to block geopathic radiation. For example, a mica-loaded electrical insulation mat may be used as sheet 20. In addition, any other sheets, mats or other layers of any material that include mica may also be used as sheets 20. In general, as the thickness of the layer of mica (or the amount of mica loaded in a particular material) in sheet 20 increases, the amount of geopathic radiation blocked by sheet 20 also increases. Although any thickness or amount of mica may be used according to the present invention, it has been observed that thicknesses of mica less than .015 inches will deteriorate within about three to four months and may need to be replaced.

As illustrated in FIGURE 1, sheets 20 may be employed to block geopathic radiation in room 10 in a variety of ways. For example, sheet 20a is positioned under a bed 30 to prevent geopathic radiation originating from under bed 30 from reaching an occupant of bed 30. Likewise, sheet 20b is placed under a chair 32 to prevent geopathic radiation originating from under chair 32 from reaching an occupant of chair 32. Although it may be more economical to only place sheets 20 under areas that are occupied for significant periods of time, such as bed 30 and chair 32, a sheet 20 may also be placed over the entire floor 36 of room 10 (thus making the entire room 10 a shielded area). Such a sheet 20 may be hidden under carpeting or placed under the surface of floor 36 during the construction of the building.

Due to reflection and refraction below the earth's surface, geopathic radiation may also propagate in a direction other than the direction that is perpendicular to the earth's surface. Such reflected or refracted radiation may travel through walls 34 of room 10 in addition to traveling through floor 36. To protect an occupant against such radiation, a sheet 20c may be hung on wall 34 to prevent geopathic radiation from traveling through wall 34. In addition, a sheet 20d may be incorporated inside wall 34 during construction and/or may be incorporated in existing thermal insulation materials.

As illustrated in FIGURE 2, sheets 20 may include one or more layers 40 of material, in addition to a layer of mica 42, that are operable to block other types of electromagnetic radiation. For example, a layer 40 of lead may be employed to block X-ray or gamma ray radiation. In addition, although not required, all of the sheets 20 described above may be fabricated such that no materials toxic to humans are included.

FIGURE 2 illustrates a building 50 having a foundation 52 that is operable to block geopathic radiation. Building 50 may be a house, apartment, hospital, office building or any other structure that may be supported by a foundation. In this embodiment, a quantity of mica is mixed into the concrete (or other material used to create the foundation 52) such that foundation 52 at least partially prevents geopathic radiation 54 from being transmitted from below surface 56 to building 50. As is illustrated in FIGURE 2, the mica in foundation 52 may absorb, diffuse, reflect, refract, or transmit geopathic radiation. Preferably, geopathic radiation is either absorbed, diffused, reflected, or refracted away from building 50. However, acceptable amounts of geopathic radiation may still be transmitted through foundation 52 to building 50.

To construct foundation 52, the concrete or other appropriate foundation material is mixed as is typical in the construction industry. At any point in this mixing process (or even when the dry concrete mix is made), a quantity of mica is mixed in the concrete or other material. This mica may be in any form, including mica powder and mica flakes. For example, but not by way of limitation, a fifty pound bag of powdered mica or mica flakes may be added to each cubic yard of concrete, or other material. The mica may be mixed into the concrete or other material in any manner such that the mica is sufficiently distributed throughout the concrete or other material. Any suitable amount of mica may be added to the concrete or other foundation material according to the present invention. However, it has been observed that the greater the quantity of mica added to the foundation, the greater amount of geopathic radiation that is blocked.

In an alternate embodiment, mica powder is added to paint during or after the paint manufacturing process. This mica powder is mixed into the paint such that the mica is suspended in the paint and at least partially prevents the transmission of geopathic radiation through the paint. The paint into which the mica is mixed may include any type of paint, including primer. The mica may be added during the manufacturing process by the paint manufacturer, during the mixing process by the paint retailer, by an end user before applying the paint, or at any other appropriate time. As described above, the greater the amount of mica used, the greater the amount of geopathic radiation that is blocked. Therefore, multiple coats of the paint may be applied to a surface to increase the amount of geopathic radiation blocked by the paint. As illustrated in FIGURE 1, the mica-paint mix 60 may be applied to walls 34 or floor 36 to at least partially prevent the transmission of geopathic radiation through these surfaces.

Mica may also be included in numerous other building materials to block the transmission of geopathic radiation into the building into which they are incorporated. For example, mica may be incorporated into or added as a layer on shingles used to roof a building, siding used to cover the sides of a building, tiles used to tile the floor of a building, linoleum flooring used to cover the floors of a building, and insulation used in the walls, floors, and ceiling of a building. Any other appropriate use of mica to block the transmission of geopathic radiation is also encompassed within the scope of the present invention.

Although the present invention has been described with several embodiments, a myriad of changes, variations, alterations, transformations, and modifications may be suggested to one skilled in the art, and it is intended that the present invention encompass such changes, variations, alterations, transformations, and modifications as fall within the spirit and scope of the appended claims.

## Claims

1. A method of blocking geopathic radiation (54), comprising:
identifying a location at which the blocking of geopathic radiation is desired; and
positioning a layer of material (20;42;52) comprising mica between a source of the geopathic radiation and the identified location, such that the geopathic radiation is at least partially prevented from being transmitted to the identified location.

2. The method of Claim 1, wherein the layer of material comprises a solid layer of mica.

3. The method of Claim 1, wherein the layer of material comprises a material loaded with mica.

4. The method of any one of Claims 1 to 3, wherein the layer of material comprises no materials toxic to humans.

5. The method of any one of Claims 1 to 4, further comprising providing a metal layer (40) coupled to the layer of material comprising mica (42), the metal layer operable to at least partially block the transmission of gamma rays.

6. The method of any one of Claims 1 to 5, further comprising providing a metal layer (40) coupled to the layer of material comprising mica (42), the metal layer operable to at least partially block the transmission of X rays.

7. The method of any one of Claims 1 to 6, wherein the source of the geopathic radiation is the core of the earth.

8. The method of any one of Claims 1 to 6, wherein the source of the geopathic radiation is a disturbance within the earth that reflects or refracts geopathic radiation emanating from the core of the earth or from another disturbance within the earth.

9. The method of any one of Claims 1 to 8, wherein positioning the layer of material between a source of geopathic radiation and the identified location comprises positioning the layer of material on a floor of a structure.

10. The method of any one of Claims 1 to 9, wherein positioning the layer of material between a source of geopathic radiation and the identified location comprises positioning the layer of material on a wall of a structure.

11. A building foundation, comprising:
a quantity of concrete sufficient to structurally support a building (50) that is to be supported by the foundation; and
a quantity of mica mixed into the concrete.

12. The building foundation of Claim 11, wherein the quantity of mica mixed into the concrete comprises a quantity of mica sufficient to at least partially prevent geopathic radiation from being transmitted from a source of geopathic radiation to the building.

13. The building foundation of Claim 11 or Claim 12, wherein the mica mixed into the concrete comprises mica powder.

14. The building foundation of any one of Claims 11 to 13, wherein the mica mixed into the concrete comprises mica flakes.

15. A method of creating a building foundation for blocking geopathic radiation, comprising:
mixing a sufficient quantity of mica into a batch of concrete such that the mica at least partially prevents geopathic radiation from being transmitted through the concrete; and
using the concrete containing the mica to create a building foundation that at least partially prevents geopathic radiation from being transmitted from a source of the geopathic radiation to a building that is supported by the foundation.

16. The method of Claim 15, wherein the mica mixed into the concrete comprises mica powder.

17. The method of Claim 15 or Claim 16, wherein the mica mixed into the concrete comprises mica flakes.

18. A method for forming a blend of mica powder and paint for blocking geopathic radiation, comprising:
adding mica powder to paint during or after the paint manufacturing process; and
mixing the mica powder into the paint such that the mica is suspended in the paint and at least partially prevents the transmission of geopathic radiation through the paint.
